# EUROPEAN PATENT SPECIFICATION

(11) **EP 2 189 129 B1**
(45) Date of publication and mention of the grant of the patent: **05.06.2013**
(21) Application number: 09275112.2
(22) Date of filing: 20.11.2009
(51) Int. Cl.: A61B 18/20

(54) **Light treatment apparatus**
Lichtbehandlungsvorrichtung
Appareil pour traitement lumineux

(30) Priority: 21.11.2008 GB 0821303
(43) Date of publication of application: 26.05.2010
(60) Divisional application: 13150182.7
(73) Proprietor: The Dezac Group Limited, Cheltenham Gloucestershire GL50 1SS (GB)
(72) Inventor: Herbert, Kevin, Cheltenham, Gloucestershire GL51 0PQ (GB); Mutton, Philip, Send, Surrey GU23 7HP (GB)
(74) Representative: Newell, William Joseph

(56) References cited:
- EP-A- 2 119 408
- WO-A-00/62700
- WO-A-2005/011510
- WO-A-2005/102201
- WO-A-2006/005443
- WO-A-2008/083305
- US-A1- 2006 020 260
- US-A1- 2007 260 230
- US-A1- 2008 091 187

## Description

Hair removal devices are disclosed in US 2008/0091187 A1 and WO 2005/102201 A1.

This invention relates to light treatment apparatus in which light is applied to the surface of the skin. In this specification the term 'light' is used to embrace devices that apply optical radiation containing several wavelengths such as intense pulsed light devices, as well as to devices which may emit radiation at a narrow waveband or single wavelength such as from so-called laser diodes. In particular, but not exclusively, the invention relates to light treatment devices for hair removal.

In a conventional hair removal process, light or laser radiation is directed to the skin to be absorbed in the hair follicle to cause the follicle to be damaged to the extent that the hair may be removed. In this specification, references to 'hair removal' and the like are used to describe a process in which sufficient energy is deposited into the hair or follicle to cause damage to allow subsequent removal or shedding of the hair whether at the treatment time or later. In such devices, in order to ensure that most of the energy deposited by the beam is absorbed in the hair follicle rather than above the skin surface, it is usual practice to advise the user to shave prior to the hair removal process. This however is time-consuming and requires the user to use different appliances first to shave and then to remove the hair.

We have however found that it is possible to use a hair removal beam initially to perform a shaving operation by directing it to pass just above the surface of the skin so that it ablates or severs the hairs in its path. We have therefore designed a laser hair treatment device which is configurable from a position in which it may be used initially to crop or shave the hair above the skin to prepare it ready for the hair removal process, to a position where it may be used as a hair remover.

Accordingly, this invention defined in the set of claims provides a hair treatment apparatus, comprising:
a housing for being placed on or adjacent the skin of a treatment subject in a given orientation:
a source of optical radiation disposed generally within said housing for emitting a beam of optical radiation;
characterised in that said apparatus is configurable between:
a cropping mode, wherein, when said housing is in its given orientation, the beam of optical radiation or a portion thereof is directed in a cropping direction to pass above and spaced from the skin of the treatment subject, in order to
provide a cropping beam to ablate or sever hairs upstanding from the skin surface, and
a hair removal mode, wherein, when said housing is in its given orientation, the beam or a portion thereof is directed in a hair removal direction towards the skin to be incident on the skin and/or on one or more hairs upstanding therefrom, in order to provide a hair removal beam to effect hair removal.

The device is reconfigured between said modes by altering the direction of the beam or a part thereof.

In a preferred arrangement, in the cropping mode, the beam of optical radiation passes closely spaced from, but parallel to the surface of the skin. With this apparatus, having first prepared the skin surface by cropping the upstanding hairs, the device can then be changed to the hair removal configuration.

There is a wide range of different ways in which a reconfigurable device may be implemented. For example the laser source may itself be movable between a position in which its output beam is aligned with the cropping direction and a position in which its output beam is aligned with the hair removal direction. However, it is preferred to provide a beam deflector which is operable to deflect a portion of the beam between the cropping direction and the hair removal direction.

Of course, two arrangements here are possible; the light source may emit the beam in the cropping direction, with the beam deflector deflecting a downstream portion of the beam from the cropping direction to the hair removal direction, or the reverse arrangement could be provided in which the source of optical radiation emits a beam in the hair removal direction and the beam deflector deflects a downstream portion of the beam from the hair removal direction to the cropping direction.

In one arrangement, the beam deflector is movable between a deflecting position, in which it deflects the beam to pass in the hair removing direction and a non-deflecting position, in which the beam extends in the cropping direction only. In this latter arrangement, the beam deflector may conveniently include an absorber surface upon which the beam is incident when the deflector is in its non-deflecting position. This prevents unwanted reflections of the beam outside the housing during the cropping operation.

Where a beam deflector is used to deflect the beam into the hair removing direction, the beam deflector is preferably movable to effect scanning of the deflected beam across the surface of the skin of a treatment subject. Scanning may be achieved by linear movement and/or angular movement.

Preferably, the apparatus includes a height adjustment arrangement for adjusting the spacing of the cropping beam from the skin of a treatment subject.

The deflector may take many forms. It may be a simple reflector or it may be a prism with an internally reflecting surface. In the latter instance, one or both of the surfaces of the prism through which the beam passes to and from the reflecting surface may be powered or have a lens attached thereto.

In the light treatment of skin, whether it be using intense pulsed light or laser radiation, and whether for hair removal or other skin treatment, it is important to ensure that the skin is not overexposed to light as this can cause skin damage or discomfort. However, in order to reduce treatment times, it is desirable to use relatively large amounts of energy. It is also desirable to be able to move the device over the surface of the skin to treat an extended area. If however a device is designed to be drawn over the skin of a user, there is a risk that the user may move the device too quickly, resulting in inadequate treatment or too slowly resulting in overexposure.

We have therefore identified a need to provide a way of ensuring that the exposure is appropriate and not excessive.

The invention may be performed in various ways, and an embodiment therefore with various modifications will now be described by way of example only, reference being made to the accompanying drawings in which:
Figure 1 is a schematic view of a hair treatment device of this invention in a hair remover mode, and
Figure 2 is a schematic view of a hair remover device of Figure 1, but in a laser shaver or cropper mode.

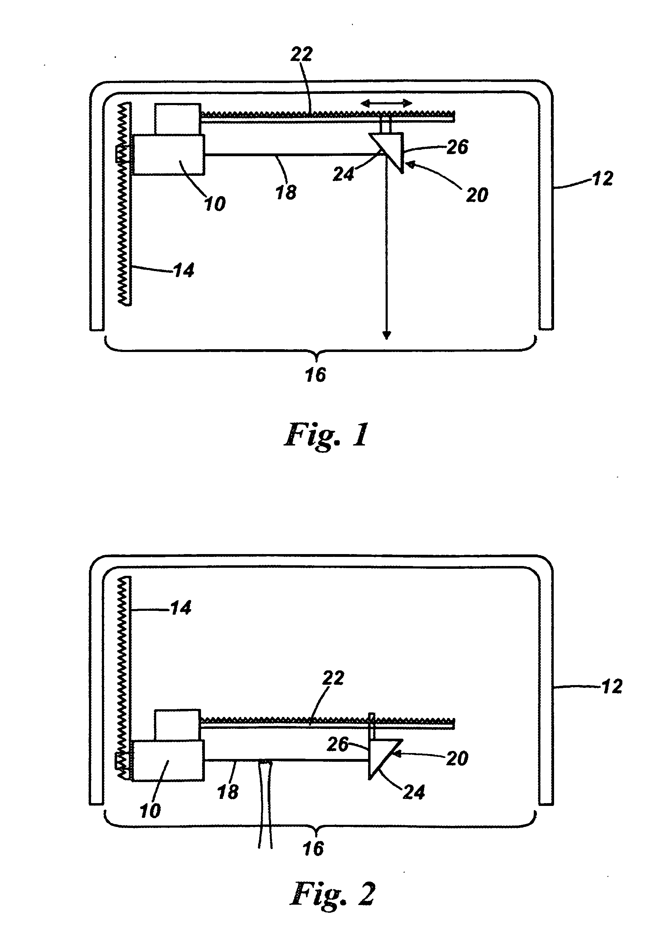

Referring initially to Figure 1, in this arrangement, a light source 10 is mounted within a housing 12 on a vertical track arrangement 14 so that the height of the light source 10 relative to a treatment aperture 16 may be varied. The light source generates a beam 18 of radiation which is capable of cropping or shaving hair by severing or ablating the hairs when the beam is in a direction parallel to the skin surface, or of destroying or damaging the hair follicles to allow hair removal if the beam is directed generally perpendicular to the skin surface. The beam may have the same intensity for both applications, or it may be adjusted between the two. The light source 10 emits a beam which passes generally parallel to the surface of the skin and then is reflected through 90° by a reflective surface 24 of a reflector 20 to pass vertically. The reflector 20 itself is mounted on a track 22 which allows it to be moved back and forth to scan the light beam across the surface of the skin. It will be appreciated that this linear scanning could also be affected by pivoting of the reflector 20.

The reflector 20 may also be pivoted to a non-reflecting position where, instead of presenting a reflecting surface 24 to the light beam it presents a nonreflective target absorber 26 (as seen in Figure 2). In this configuration, the light source 10 and reflector 20 can be brought close to the surface of the skin so the light beam passes parallel to but spaced just above the surface of the skin to shave or crop the hairs on which it is incident. This movement is achieved by means of the height adjuster mechanism 14.

The height adjuster mechanism 14 allows the height to be set at different heights according to the grade of cut required. Also, if required, the height adjuster could adjust the height dynamically as the device is drawn across the surface of the skin so as to follow skin contours. For this purpose a height detector may be provided which may operate using optical or other suitable technologies. This be done in a variety of different ways, for example by using a reflected pulsed light beam possibly (using either the reflected time shift, or a Doppler shift, to determine distance). Alternatively, sound could be used to determine distance using echo measurements. Most likely however is the whole laser head could be mounted so as to move with the skin (similar to a razor head being tilted and movable) so that the distance is always fixed. Finally, a camera monitor detail on the skin and compare sizes in successive frames and determine the distance accordingly. Alternatively for hair removal, a camera and controller could monitor the size of the beam spot - as the distance changed the spot would get larger as the beam is divergent.

In the above arrangement, the reflector 20 is a mirrored external surface. It will be appreciated that this of course could be an internal reflecting surface of a prism. In this instance, the inlet and/or outlet surfaces of the prism may be powered and/or have a lens attached thereto for focussing or other beam shaping functions.

In use, the user will first set the device to shaver or crop mode as seen in Figure 2, drawing the device across their skin as the light source is energised to cut the hair close to the skin. Having done this and cleared away the cut hair and other debris, the device is switched to the configuration of Figure 1 by flipping the reflector 20 and moving the mechanism away from the skin if required by the height adjuster. The light source can then be used to effect laser hair removal treatment of the cropped/shaved follicles.

## Claims

1. A hair treatment apparatus, comprising:
a housing (12) for being placed on or adjacent the skin of a treatment subject in a given orientation;
a source (10) of optical radiation disposed generally within said housing for emitting a beam of optical radiation;
**characterised in that** said apparatus is configurable between:
a cropping mode, wherein, when said housing is in said given orientation, the beam of optical radiation or a portion thereof is directed In a cropping direction to pass above and spaced from the skin of the treatment subject in order to provide a cropping beam to ablate or sever hairs upstanding from the skin surface, and
a hair removal mode, wherein, when said housing is in said given orientation, the beam or a portion thereof is directed in a hair removing direction towards the skin to be incident on the skin and/or on one or more hairs upstanding therefrom in order to provide a hair removal beam to facilitate hair removal.

2. A hair treatment apparatus according to Claim 1, comprising a beam deflector (20) operable to deflect said beam between said cropping direction and said hair removing direction.

3. A hair treatment apparatus according to Claim 1 or Claim 2, wherein said source of optical radiation (10) emits a beam generally in said cropping direction.

4. A hair treatment apparatus according to Claim 1 or Claim 2, wherein said source of optical radiation (10) emits a beam generally in said hair removing direction.

5. A hair treatment apparatus according to Claim 2 or Claim 3 referred back to claim 2, wherein said beam deflector (20) is movable between a deflecting position, in which it deflects said beam to pass in said hair removing direction, and a non-deflecting position in which said beam extends in said cropping direction only.

6. A hair treatment apparatus according to Claim 5, wherein said beam deflector (20) includes an absorbent surface (26) upon which said beam is incident when the deflector is in its non-deflecting position.

7. A hair treatment apparatus according to Claim 2 or Claim 3 referred back to claim 2, wherein said beam deflector (20) is movable to scan said deflected beam across the surface of the skin of a treatment subject.

8. A hair treatment apparatus according to any of the preceding Claims, including a height adjustment arrangement (14) for adjusting the spacing of the cropping beam from the skin of a treatment subject in use.

## Patentansprüche

1. Haarbehandlungs-Vorrichtung, umfassend ein Gehäuse (12), das auf oder an der Haut eines Behandlungs-Gegenstandes in einer gegebenen Ausrichtung angeordnet wird; eine Quelle (10) optischer Strahlung, die im allgemeinen innerhalb des Gehäuses angeordnet ist und dazu dient, einen Strahl der optischen Strahlung auszusenden, **dadurch gekennzeichnet, daß** die Vorrichtung zwischen einem Schneidmodus und einem Haarentfernungsmodus einstellbar ist, wobei in dem Schneidmodus dann, wenn das Gehäuse eine gegebene Ausrichtung einnimmt, der Strahl der optischen Strahlung oder ein Teil davon in einen Schneidmodus gelenkt wird, um sich über der Haut des behandelten Gegenstandes und mit Abstand von ihr getrennt zu bewegen und dadurch einen Schneidstrahl zu erzeugen, um hochstehende Haare von der Hautoberfläche zu entfernen oder abzuschneiden, und wobei in dem Haarentfernungsmodus dann, wenn das Gehäuse sich in der gegebenen Ausrichtung befindet, der Strahl oder ein Teil des Strahles in eine Haarentfernungs-Richtung zu der Haut gelenkt werden, so daß er auf der Haut auftrifft und / oder auf einem oder mehreren Haaren, die von der Haut nach oben ragen, um dadurch einen Haarentfernungsstrahl zu erzeugen, der das Entfernen des Haares erleichtert.

2. Haarbehandlungs-Vorrichtung nach Anspruch 1, **gekennzeichnet durch** eine Strahlablenk-Einrichtung (20), die so arbeitet, daß sie den Strahl zwischen der Schneidrichtung und der Haarentfernungs-Richtung lenkt.

3. Haarbehandlungs-Vorrichtung nach Anspruch 1 oder 2, **dadurch gekennzeichnet, daß** die Quelle der optischen Strahlung (10) einen Strahl im allgemeinen in der besagten Schneidrichtung aussendet.

4. Haarbehandlungs-Vorrichtung nach Anspruch 1 oder 2, **dadurch gekennzeichnet, daß** die Quelle der optischen Strahlung (10) einen Strahl im allgemeinen in der Haarentfernungs-Richtung aussendet.

5. Haarbehandlungs-Vorrichtung nach Anspruch 2 oder 2 und 3, **dadurch gekennzeichnet, daß** die Strahlablenk-Einrichtung (20) zwischen einer Ablenkposition, in der sie den Strahl ablenkt, so daß er sich in Haarentfernungs-Richtung befindet, bewegbar ist sowie einer nicht ablenkenden Position, in der sich der Strahl nur in der Schneidrichtung erstreckt.

6. Haarbehandlungs-Vorrichtung nach Anspruch 5, **dadurch gekennzeichnet, daß** die Strahlablenk-Einrichtung eine absorbierende Oberfläche (26) aufweist, auf die der Strahl auftrifft, wenn sich die Ablenkeinrichtung in ihrer nicht ablenkenden Position befindet.

7. Haarbehandlungs-Vorrichtung nach Anspruch 2 oder 3 und 2, **dadurch gekennzeichnet, daß** die Strahlablenk-Einrichtung (20) so bewegbar ist, daß der abgelenkte Strahl die Oberfläche der Haut eines behandelten Gegenstandes abtastet.

8. Haarbehandlungs-Vorrichtung nach einem der vorhergehenden Ansprüche, **gekennzeichnet durch** eine Höheneinstell-Anordnung (14), die dazu dient, den Abstand des schneidenden Strahls von der Haut eines behandelten Gegenstandes bei Benutzung einzustellen.

## Revendications

1. Appareil de traitement des poils, comprenant :
- un boîtier (12) destiné à être placé sur ou au voisinage de la peau d'un sujet de traitement dans une orientation donnée ;
- une source (10) de rayonnement optique disposée généralement à l'intérieur dudit boîtier pour émettre un faisceau de rayonnement optique ;
**caractérisé par le fait que** ledit appareil est apte à être configuré entre :
un mode de coupe, dans lequel, lorsque ledit boîtier est dans ladite orientation donnée, le faisceau de rayonnement optique ou une partie de celui-ci est dirigé dans une direction de coupe pour passer au-dessus et espacé de la peau du sujet de traitement de façon à fournir un faisceau de coupe pour réaliser l'ablation ou la séparation des poils se dressant à partir de la surface de peau ; et
un mode de retrait de poils, dans lequel, lorsque ledit boîtier est dans ladite orientation donnée, le faisceau ou une partie de celui-ci est dirigé dans une direction de retrait de poils vers la peau pour être incident sur la peau et/ou sur un ou plusieurs poils se dressant à partir de celle-ci de façon à fournir un faisceau de retrait de poils pour faciliter le retrait de poils.

2. Appareil de traitement des poils selon la revendication 1, comprenant un déflecteur de faisceau (20) pour dévier ledit faisceau entre ladite direction de coupe et ladite direction de retrait de poils.

3. Appareil de traitement des poils selon la revendication 1 ou la revendication 2, dans lequel ladite source de rayonnement optique (10) émet un faisceau généralement dans ladite direction de coupe.

4. Appareil de traitement des poils selon la revendication 1 ou la revendication 2, dans lequel ladite source de rayonnement optique (10) émet un faisceau généralement dans ladite direction de retrait de poils.

5. Appareil de traitement des poils selon la revendication 2 ou la revendication 3 dans la mesure où elle se réfère à la revendication 2, dans lequel ledit déflecteur de faisceau (20) est mobile entre une position de déviation, dans laquelle il dévie ledit faisceau pour le faire passer dans ladite direction de retrait de poils, et une position de non déviation dans laquelle ledit faisceau s'étend dans ladite direction de coupe seulement.

6. Appareil de traitement des poils selon la revendication 5, dans lequel ledit déflecteur de faisceau (20) comprend une surface absorbante (26) sur laquelle ledit faisceau est incident lorsque le déflecteur est dans sa position de non-déviation.

7. Appareil de traitement des poils selon la revendication 2 ou la revendication 3 dans la mesure où elle se réfère à la revendication 2, dans lequel ledit déflecteur de faisceau (20) est mobile pour balayer ledit faisceau dévié à travers la surface de la peau d'un sujet de traitement.

8. Appareil de traitement des poils selon l'une quelconque des revendications précédentes, comprenant un agencement d'ajustement en hauteur (14) pour ajuster l'espacement du faisceau de coupe à partir de la peau d'un sujet de traitement en utilisation.
